# EUROPÄISCHE PATENTSCHRIFT

(11) **EP 2 434 778 B1**
(45) Veröffentlichungstag und Bekanntmachung des Hinweises auf die Patenterteilung: **27.07.2016**
(21) Anmeldenummer: 11007416.8
(22) Anmeldetag: 13.09.2011
(51) Int. Cl.: A61F 2/18, H04R 25/00

(54) **Anordnung zur längenvariablen Fixierung des Aktor-Endstücks eines aktiven Hör-Implantats im Mittelohr**
Assembly for variable length fixation of the actuator end piece of an active hearing implant in the inner ear
Agencement de fixation variable en longueur de l'extrémité d'actionneur d'un implant auditif actif dans l'oreille moyenne

(30) Priorität: 24.09.2010 DE 102010046457
(43) Veröffentlichungstag der Anmeldung: 28.03.2012
(73) Patentinhaber: Heinz Kurz GmbH Medizintechnik, 72144 Dusslingen (DE)
(72) Erfinder: Lenarz, Thomas, Prof.,Dr.,med., 30559 Hannover (DE); Steinhardt, Uwe, 72145 Hirrlingen (DE); Kurz, Heinz, 72144 Dusslinden (DE)
(74) Vertreter: Kohler Schmid Möbus Patentanwälte

(56) Entgegenhaltungen:
- DE-A1-102005 027 215
- DE-U1- 20 014 659
- DE-U1-202005 011 485

## Beschreibung

Die Erfindung betrifft eine Anordnung zum Einstellen und Fixieren der Relativlage zwischen einerseits einem zylinderförmigen Aktor-Endstück eines aktiven Hör-Implantats und andererseits einem Glied der Gehörknöchelchen-kette oder einem Ankoppelteil zum menschlichen Innenohr, insbesondere zur Perilymphe des labyrinthischen Systems oder zum Endolymphraum, wobei die Anordnung ein Verbindungselement mit einem ersten Ankoppelelement zum Ankoppeln des Verbindungs-elements an das Aktor-Endstück, mit einem zweiten Ankoppelelement zum Ankoppeln des Verbindungselements an den Amboss, den Steigbügel, die Steigbügelfußplatte, das ovale Fenster oder das runde Fenster des menschlichen Mittelohrs sowie mit einem schaftförmigen Mittelteil zwischen dem ersten Ankoppelelement und dem zweiten Ankoppelelement umfasst.

Eine solche Anordnung zum Einstellen und Fixieren der Relativlage zweier Elemente eines aktiven Hör-Implantats ist bekannt aus der US-A 5,941,814.

Hör-Implantate werden verwendet, um bei ganz oder teilweise fehlenden oder geschädigten Gehörknöchelchen des menschlichen Mittelohrs den auf die Ohrmuschel treffenden Schall bzw. ein entsprechendes Schallsignal zum Innenohr zu übertragen. Man unterscheidet dabei zwischen passiven Gehörknöchelchenprothesen einerseits, die Teile der Gehörknöchelchenkette physisch ersetzen, wobei die Schallleitung "passiv", also ohne Zuhilfenahme von elektronischen Hilfsmitteln erfolgt, und aktiven Hör-Implantaten andererseits, die den Schallsignalen entsprechende elektrische Signale aus einem elektronischen Verstärker eines außerhalb des Mittelohrs angebrachten Hörgeräts mittels eines im Mittelohr implantierten Aktors empfangen, dort durch mechanische Bewegung wieder in akustische Schwingungen umsetzen und von einem vibrierenden Aktor-Endstück über ein geeignetes Verbindungselement in das Innenohr übertragen. Derartige aktive Hör-Implantate und insbesondere die Problematik einer optimalen akustischen Ankopplung des Aktor-Endstücks an das Innenohr sind Gegenstand der vorliegenden Erfindung.

Der Durchmesser des in der Regel zylinderförmigen Aktor-Endstücks ist normalerweise genormt, also immer von gleicher Größe oder zumindest in einer Gruppe bestimmter diskreter Größen zur Auswahl für unterschiedliche individuelle Gegebenheiten beim jeweiligen Patienten vorhanden.

Aus der DE 200 14 659 U1 ist eine Anordnung zum Einstellen und Fixieren der Relativlage zwischen dem Aktor-Endstück ("Aktuator") eines aktiven Hörimplantats einerseits und einem Glied der menschlichen Gehörknöchelchenkette anderseits bekannt. Dabei weist die bekannte Anordnung ein Verbindungselement mit einem ersten Ankoppelelement in Form einer Steckhülse mit Klammern zum Ankoppeln an das Aktor-Endstück, mit einem zweiten Ankoppelelement in Form eines Clips zum Ankoppeln an den Steigbügel sowie mit einem schaftförmigen Mittelteil zwischen dem ersten und dem zweiten Ankoppelelement auf.

In der DE 10 2005 027 215 A1 wird beschrieben, dass es "theoretisch auch denkbar ist, dass Feder- oder Rastelemente an dem Piston an oder in der Bohrung vorgesehen sind, die dazu ausgestaltet sind, um mit entsprechenden Gegenmitteln einzugreifen (z.B. Rastvertiefungen), die an der Umfangsfläche des Schafts ausgebildet sind". Damit soll wohl eine längenvariable Anpassung der Stapesprothese ermöglicht werden.

In der eingangs zitierten US-A 5,941,814 wird als erstes Ankoppelelement zum Ankoppeln des Verbindungselements an das Aktor-Endstück eine als hohler Zylinder ausgebildete Crimphülse vorgeschlagen, durch welche das Aktor-Endstück während der Implantationsoperation gesteckt und anschließend mittels eines Crimpwerkzeugs durch Kaltverformung dauerhaft befestigt wird. Zwischen der Außenseite dieser Crimphülse und dem schaftförmigen Mittelteil besteht eine starre mechanische Verbindung, etwa durch einen Schweiß- oder Lötpunkt.

Problematisch ist bei dieser bekannten geometrischen Anordnung die fehlende Flexibilität für eine exakte räumliche Relativ-Positionierung zwischen dem vibrierenden Aktor-Endstück und dem ersten Ankoppelelement des Verbindungselements im Mittelohr. So kann zwar durch ein weiteres oder weniger weites Hineinschieben des ersten Ankoppelelements in den zylindrischen Hohlraum der Crimphülse eine gewisse Variabilität in Richtung der Zylinderachse der Crimphülse erreicht werden. Eine Einstellmöglichkeit in einer Richtung senkrecht zu dieser Zylinderachse beseht jedoch überhaupt nicht, so dass die FeinPositionierung der Verbindungsstelle zwischen dem Aktor-Endstück und dem ersten Ankoppelelement im Raum und damit eine genaue Fixierung der endgültigen Lage der gesamten Anordnung nur höchst ungenau erfolgen kann.

Dies führt entsprechend zu ungewünschten Verspannungen innerhalb des Implantat-Aufbaus sowie zu suboptimaler geometrischer Anpassung der Relativlagen der einzelnen Anschlussteile, was letztlich wiederum einen deutlich schlechteren Frequenzgang des gesamten Hör-Implantats und eine geringere Verbesserung der Schallleitung zur Folge hat, als die Gesamtanordnung mit dem aktiven Hörgerät technisch eigentlich leisten könnte.

Aufgabe der vorliegenden Erfindung ist es demgegenüber, mit möglichst einfachen technischen Mitteln unaufwändig und kostengünstig eine gattungsgemäße Anordnung der eingangs beschriebenen Art bereit zu stellen, die zusätzlich zu der Verstellbarkeit in Achsrichtung des Aktor-Endstücks auch eine bequeme längenvariable Einpassung und Fixierung des Aktor-Endstücks relativ zum ersten Ankoppelelement in einer Richtung quer zur Achse des Aktor-Endstücks ermöglicht, so dass im Endeffekt die vom Aktor erzeugten akustischen Schwingungen in Form von Schallwellen besser an die zum Innenohr führenden Teile des Hör-Implantats weitergeleitet werden können.

Erfindungsgemäß wird diese Aufgabe auf ebenso überraschend einfache wie wirkungsvolle Weise dadurch gelöst, dass das erste Ankoppelelement geometrisch so gestaltet ist, dass es das zylinderförmige Aktor-Endstück mit zwei gegenüber liegenden, im Wesentlichen parallelen Schenkeln derart umgreift, dass zusätzlich zu der Verstellbarkeit in Achsrichtung des zylinderförmigen Aktor-Endstücks auch eine bequeme längenvariable Einpassung und Fixierung des Aktor-Endstücks relativ zum ersten Ankoppelelement in einer Richtung quer zur Achse des zylinderförmigen Aktor-Endstücks ermöglicht wird, und dass die beiden Schenkel jeweils eine Rastereinrichtung aufweisen, die im Zusammenwirken der beiden Schenkel ermöglicht, das umgriffene Aktor-Endstück auf wählbaren diskreten axialen Positionen in einer Richtung parallel zur Schaftachse des schaftförmigen Mittelteils zu fixieren.

Wenn die diskreten axialen Klemmpositionen zwischen den beiden Schenkeln geometrisch fein genug abgestuft sind, lässt sich damit eine äußerst exakte räumliche Einstellung des Ankoppelpunkts zwischen Aktor-Endstück und erstem Ankoppelelement bewirken, welche andererseits auch die Möglichkeit der räumlichen Positionierung der gewünschten Lage des zweiten Ankoppelelements im Mittelohr erheblich verbessert.

Besonders bevorzugt und relativ einfach herzustellen ist eine Klasse von Ausführungsformen der erfindungsgemäßen Anordnung, bei denen die Rastereinrichtungen der Schenkel des ersten Ankoppelelements auf den im implantierten Zustand am Außenumfang des Aktor-Endstücks anliegenden Innenflächen jeweils an gegenüberliegenden axialen Positionen der beiden Schenkel Einkerbungen oder Einbuchtungen zur Aufnahme des Aktor-Endstücks aufweisen. Damit lässt sich die gewünschte Einstellung der axialen Endposition besonders genau und reproduzierbar bewerkstelligen.

Eine vorteilhafte Weiterbildung in dieser Klasse von Ausführungsformen zeichnet sich dadurch aus, dass die Einkerbungen oder Einbuchtungen auf den Innenflächen der beiden Schenkel jeweils in axialer Richtung parallel zur Schaftachse des schaftförmigen Mittelteils äquidistant angeordnet sind, was auch fertigungstechnisch besonders günstig ist.

Um eine besonders gute und dauerhaft stabile Passung der Anordnung nach dem Einstellen der gewünschten relativen Endposition von Aktor-Endstück und erstem Verbindungselement zu gewährleisten, weist bei bevorzugten Weiterbildungen dieser Klasse von Ausführungsformen das Aktor-Endstück auf seinem Außenumfang Ausbuchtungen auf, die geometrisch so gestaltet sind, dass sie als Gegenstücke zu den Einkerbungen oder Einbuchtungen an den diskreten axialen Positionen der Rastereinrichtungen des ersten Ankoppelelements passen.

Alternativ oder ergänzend zeichnet sich eine bevorzugte Weiterbildung dieser Klasse von Ausführungsformen dadurch aus, dass das Aktor-Endstück auf seinem Außenumfang azimutal verlaufende Ringnuten aufweist, die geometrisch so gestaltet sind, dass sie als Gegenstücke zu den Einkerbungen oder Einbuchtungen an den diskreten axialen Positionen der Rastereinrichtungen des ersten Ankoppelelements passen. Damit kann insbesondere bei Verwirklichung mehrerer in axialer Richtung des Aktor-Endstücks hintereinander angeordneter Ringnuten eine genaue Positionierung des ersten Ankoppelelements relativ zum Aktor-Endstück in dessen Axialrichtung bewirkt werden.

Sowohl im Hinblick auf eine technisch leichte Herstellbarkeit als auch auf eine besonders einfache Handhabung ist eine weitere Klasse von Ausführungsformen der erfindungsgemäßen Anordnung zu bevorzugen, bei welcher der erste Schenkel des ersten Ankoppelelements in den schaftförmigen Mittelteil des Verbindungselements übergeht, der zweite Schenkel über ein Verbindungsstück in seitlichem Abstand mit dem ersten Schenkel verbunden ist, und an dem dem Verbindungsstück entgegengesetzten Ende zwischen dem ersten Schenkel und dem freien Ende des zweiten Schenkels ein Einführbereich zum Einführen des Aktor-Endstücks vorgesehen ist.

Für eine weitere Verbesserung der Handhabung sorgen Weiterbildungen dieser Klasse von Ausführungsformen, bei welchen das freie Ende des zweiten Schenkels in eine vom ersten Schenkel weg in Richtung auf die Außenseite des zweiten Schenkels schräg nach außen verlaufende Einführhilfe für das Einsetzen des Aktor-Endstücks in den Einführbereich des ersten Ankoppelelements mündet.

Vorteilhaft und leicht herstellbar sind auch Weiterbildungen, bei welchen das Verbindungsstück am freien, dem schaftförmigen Mittelteil entgegengesetzten Ende des ersten Ankoppelelements angeordnet ist.

Zur Erhöhung der Formsteifigkeit des Verbindungselements im bereich des ersten Ankoppelelements sowie für eine verbesserte Handhabung während der Implantation dienen Weiterbildungen dieser Klasse von Ausführungsformen, bei denen das Verbindungsstück zwei Hörner aufweist, die seitlich jeweils in einer Richtung von der Schaftachse des schaftförmigen Mittelteils weg nach außen ragen.

Nachdem das Hör-Implantat operativ im Mittelohr platziert wurde, beginnt die so genannte Einheilphase. In dieser Zeit bilden sich Narben und diese verursachen unvorhersehbar Kräfte, welche dazu führen können, die Prothese aus ihrer lokalen, während der Operation exakt eingestellten Position zu verschieben. Bei einer zu steifen Verbindung zwischen dem ersten und dem zweiten Ankoppelelement kann es außerdem zu erhöhten Druckspitzen kommen, die wiederum zu Beschädigungen des Implantats führen können. Aus diesem Grund ist es sehr hilfreich, wenn dieser Teil des aktiven Hör-Implantats eine gewisse post-operative Mobilität aufweist, so dass er sich postoperativ selbstständig einer veränderten Position in den Randbereichen angleichen kann. Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Hör-Implantate nicht starr ausgebildet sind, sondern eine gewisse Flexibilität oder Variabilität aufweisen.

In der Regel wird bei der erfindungsgemäßen Anordnung das Mittelteil zwischen den beiden Befestigungselementen als länglicher Schaft gestaltet sein, wie dies an sich aus dem Stand der Technik wohlbekannt ist. Um die oben erörterte erhöhte Flexibilität bzw. Variabilität der Prothese zu erreichen - wie beispielsweise ausführlich in der EP 1 181 907 B1 beschrieben - kann bei einer besonders bevorzugten Klasse von Ausführungsformen der erfindungsgemäßen Anordnung das Verbindungselement ein Kugelgelenk aufweisen, die insbesondere im Bereich des schaftförmigen Mittelteils angeordnet ist. Dadurch wird dem Verbindungselement eine gewisse Flexibilität verliehen. Vorteilhaft im Hinblick auf eine besonders hohe postoperative Beweglichkeit des Verbindungselements sind Weiterbildungen, bei denen der längliche Schaft eine Vielzahl von aneinander angrenzenden weiteren Drehelementen, vorzugsweise eine Kugelgelenkkette, umfasst.

Alternativ kann bei einer Klasse von besonders einfach herstellbaren Ausführungsformen der erfindungsgemäßen Anordnung das erste Ankoppelelement und das schaftförmige Mittelteil einstückig aufgebaut sein.

Fertigungstechnisch besonders günstig sind Weiterbildungen dieser Klasse von einfachen Ausführungsformen, bei welchen das Verbindungselement mit erstem Ankoppelelement, schaftförmigem Mittelteil und zweitem Ankoppelelement insgesamt einstückig aufgebaut ist.

Möglich sind Ausführungsformen der Erfindung, bei denen die Anordnung oder Teile davon aus biokompatiblen Kunststoffen, insbesondere Silikon, Polytetrafluorethylen (PTFE) oder Polyetheretherketon (PEEK), und/oder aus Faserverbundwerkstoffen, insbesondere Kohlefasern hergestellt sind. Mit diesen Materialien können postoperative Abstoßungsreaktionen in den meisten Fällen verhindert werden.

Die erfindungsgemäße Anordnung oder Teile davon können aber auch aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen das Verbindungselement ganz oder teilweise zumindest im Bereich des ersten Ankoppelelements aus einem Material mit Formgedächtnis (=memory effect) oder superelastischen Eigenschaften, insbesondere aus einer Nickel-Titan-Legierung, vorzugsweise aus Nitinol hergestellt ist, was per se beispielsweise aus der WO 02/069850 A1 oder der US 6,554,861 B2 - allerdings nur im Zusammenhang mit passiven Gehörknöchelchenprothesen - bekannt ist.

Alternativ oder ergänzend können bei weiteren Ausführungsformen Teile der erfindungsgemäßen Anordnung aus einem Keramikmaterial hergestellt sein.

Weitere bevorzugte Ausführungsformen der erfindungsgemäßen Anordnung zeichnen sich dadurch aus, dass das zweite Ankoppelelement als Piston oder Kugel zur Ankopplung an das Innenohr oder als Clip oder als Glocke zur Ankopplung an das Steigbügel-Köpfchen oder als Aufnahmeteil für eine Ω-förmige künstliche Steigbügelfußplatte oder als Stempel zur Ankopplung an die natürliche Steigbügelfußplatte oder als Klammer zur Ankopplung an den Incus-Fortsatz oder als Zapfen zur Ankopplung an den Incus-Körper ausgebildet ist. Verschiedene Möglichkeiten zur Ausgestaltung des zweiten Ankoppelelements als Klammer zur Befestigung am Incus-Fortsatz sind an sich in US-A 5,935,167, in US 6,830,587 B2 und in US 7,628,812 B2 beschrieben, während die US 2008/0208338 A1 eine speziell ausgestaltete Schlinge für die mechanische Ankopplung an ein anderes Glied der menschlichen Gehörknöchelchenkette vorschlägt. Darüber hinaus beschreibt etwa die DE 10 2009 016 468 B3 Ausgestaltungen des zweiten Befestigungselements als Kolben (="Piston"), Stempel oder geschlitzte Glocke. Allerdings handelt es sich bei sämtlichen dieser bekannten Ankoppelelemente jeweils nicht um Teile von gattungsgemäßen aktiven Hör-Implantaten, sondern vielmehr von gattungsfremden passiven Gehörknöchelchenprothesen, die zudem keinerlei Hinweis auf die erfindungsgemäße geometrische Gestaltung des ersten Ankoppelelements zu geben vermögen.

Neben der weiter oben beschriebenen postoperativen Positionsverschiebung ergibt sich nach der Implantation von Hör-Implantaten auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation eines Hör-Implantats immer auch eine möglichst lange, komplikationsfreie Verweildauer des Implantats im Mittelohr des Patienten sein muss, kann ein lange andauernder Materialangriff zu Beschädigungen des Implantats und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Anordnung ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Ein direkt ins Innenohr führendes, etwa in Form eines Kolbens ausgebildetes zweites Befestigungselement wird beispielsweise eine wachstumshemmende Beschichtung aufweisen, um eine ungewünschte Versteifung und daraus folgende Unbeweglichkeit zu verhindern. Besonders bevorzugt schließlich ist eine Ausführungsform der erfindungsgemäßen Anordnung, bei der die Massenverteilung der einzelnen Teile des Hör-Implantats in Abhängigkeit von einem gewünschten, vorgegebenen oder vorgebbaren Frequenzgang der Schallleitung im Mittelohr berechnet ist. Damit lässt sich ohne großen zusätzlichen technischen Aufwand gewissermaßen ein mechanisches Tuning der Schallfortpflanzungseigenschaften mittels eines individuellen ausgestalteten Hör-Implantats erreichen.

Ein solcher Tuning-Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr am Verbindungselement befestigt ist. Bei vorteilhaften Weiterbildungen dieser Ausführungsformen ist die zusätzliche Masse mittels eines Clips an einem Teil der Gehörknöchelchenkette oder des Hör-Implantats befestigt. Außerdem können die zusätzliche Masse und/oder der Clip ebenfalls mit einer biologisch aktiven Beschichtung überzogen sein.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Im Einzelnen zeigen:
- Fig. 1a: eine schematische räumliche Darstellung einer flexiblen Ausführungsform der erfindungsgemäßen Anordnung mit zwei parallelen axial verlaufenden Ausbuchtungen am Aktor-Endstück, mit einem Kugelgelenk im schaftförmigen Mittelteil und mit einem Aufnahmeteil für eine Ω-förmige künstliche Steigbügelfußplatte als zweitem Ankoppelelement;
- Fig. 1b: wie Fig. 1a, jedoch mit drei parallel angeordneten azimutal verlaufenden Ringnuten am Außenumfang des Aktor-Endstücks;
- Fig. 2: eine schematische räumliche Darstellung einer einfachen Ausführungsform des erfindungsgemäßen Verbindungselements mit durchgehendem schaftförmigen Mittelteil und einem Piston als zweitem Ankoppelelement;
- Fign. 3a,b: eine schematische Detailansicht eines
(a) als Piston
(b) als Kugel ausgeführten zweiten Ankoppelelements zur direkten Ankopplung des Verbindungselements an das Innenohr;
- Fign. 4a,b: eine schematische Detailansicht eines
(a) als Clip
(b) als geschlitzte Glocke ausgeführten zweiten Ankoppelelements zur Ankopplung des Verbindungselements zur Ankopplung an das Steigbügel-Köpfchen;
- Fig. 5: eine schematische Detailansicht eines als Aufnahmeteil für eine Ω-förmige künstliche Steigbügelfußplatte ausgeführten zweiten Ankoppelelements zur Ankopplung des Verbindungselements an die künstliche Steigbügelfußplatte;
- Fig. 6: eine schematische Detailansicht eines als Stempel ausgeführten zweiten Ankoppelelements zur Ankopplung des Verbindungselements an die natürliche Steigbügelfußplatte;
- Fig. 7: eine schematische Detailansicht eines als Klammer ausgeführten zweiten Ankoppelelements zur Ankopplung des Verbindungselements an den Incus-Fortsatz; und
- Fig. 8: eine schematische Detailansicht eines als Zapfen ausgeführten zweiten Ankoppelelements zur Ankopplung des Verbindungselements an den Incus-Körper.

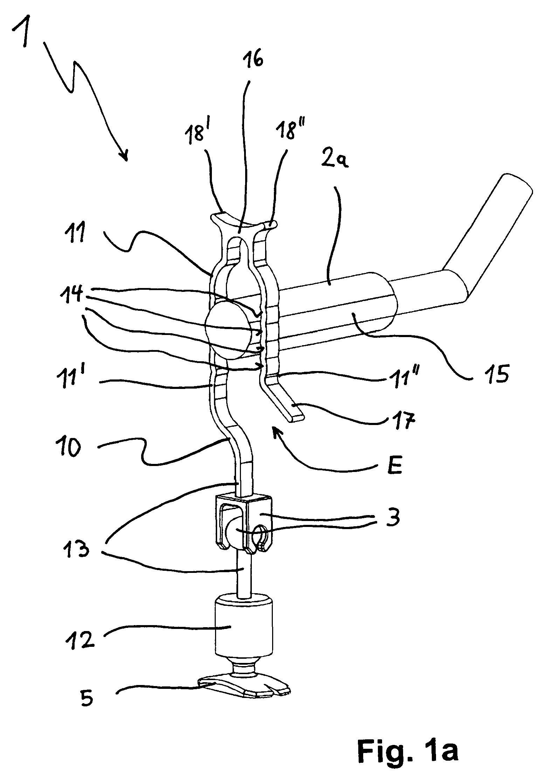

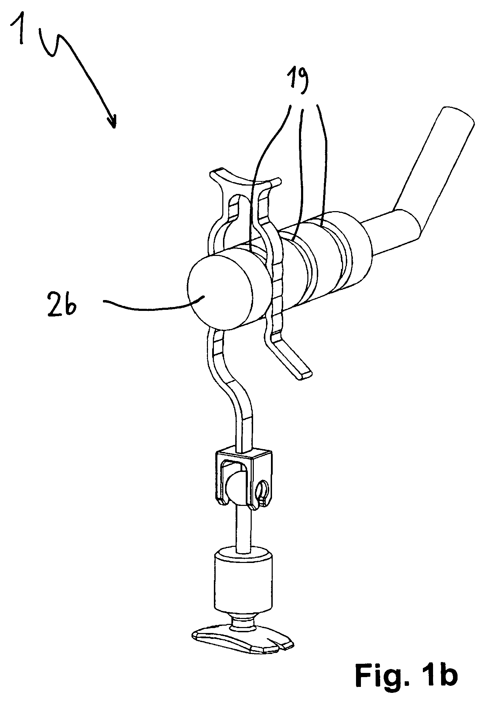

Die in den Figuren 1a und 1b schematisch räumlich dargestellten Ausführungsformen der erfindungsgemäßen Anordnung 1 dienen zum Einstellen und Fixieren der Relativlage zwischen einerseits einem Aktor-Endstück 2a; 2b eines - ansonsten in der Zeichnung nicht näher gezeigten - aktiven Hör-Implantats und andererseits einem Glied der Gehörknöchelchenkette oder einem Ankoppelteil zum menschlichen Innenohr, insbesondere zur Perilymphe des labyrinthischen Systems oder zum Endolymphraum.

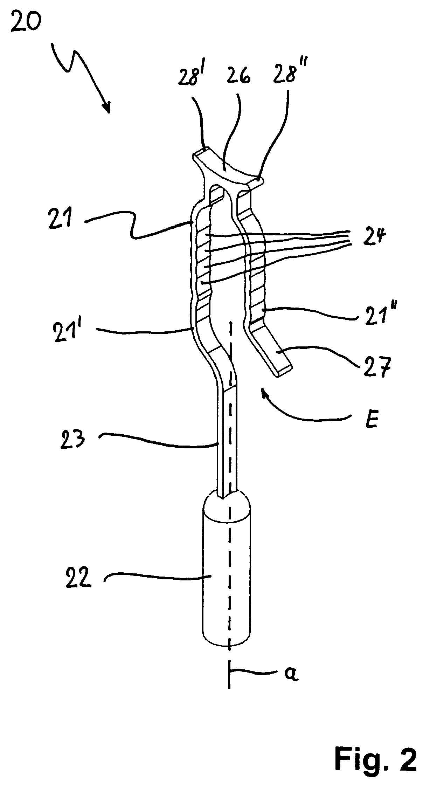

Die Anordnung 1 umfasst ein - in einer weiteren Ausführungsform auch in Fig. 2 dargestelltes - Verbindungselement 10; 20 mit einem ersten Ankoppelelement 11; 21 zum Ankoppeln des Verbindungselements 10; 20 an das Aktor-Endstück 2a; 2b, mit einem zweiten Ankoppelelement 12; 22 zum Ankoppeln des Verbindungselements 10; 20 an den Amboss, den Steigbügel, die Steigbügelfußplatte, das ovale Fenster oder das runde Fenster des menschlichen Mittelohrs sowie mit einem schaftförmigen Mittelteil 13; 23 zwischen dem ersten Ankoppelelement 11; 21 und dem zweiten Ankoppelelement 12; 22.

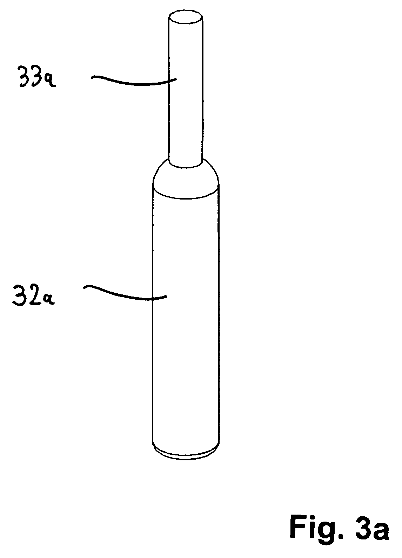

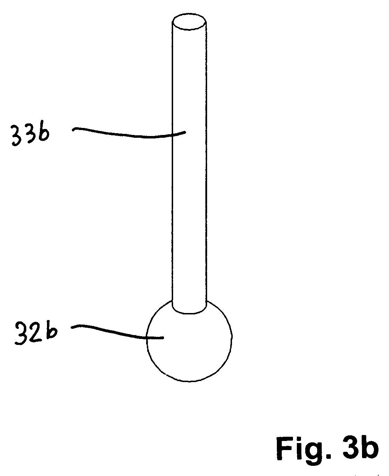

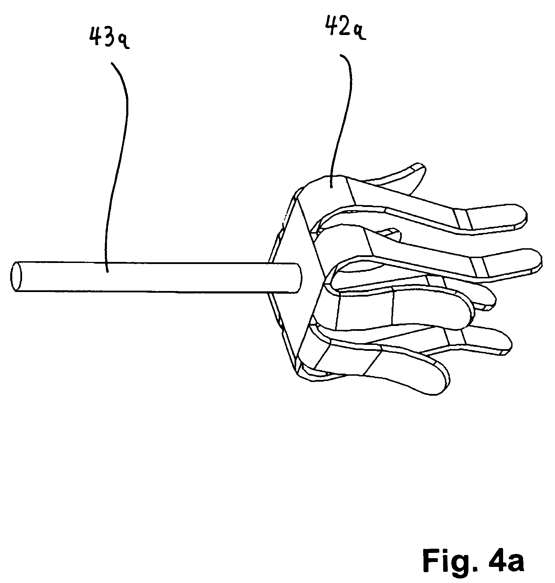

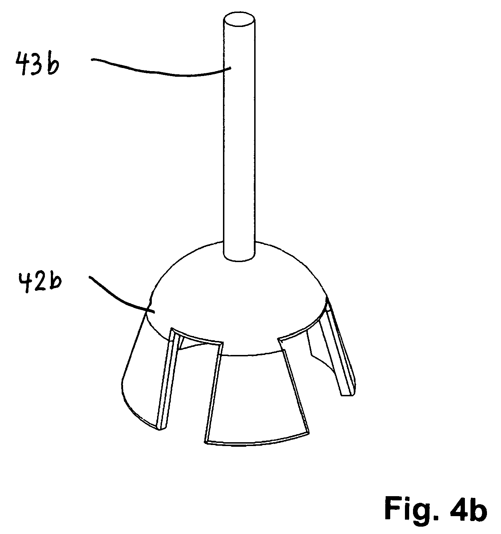

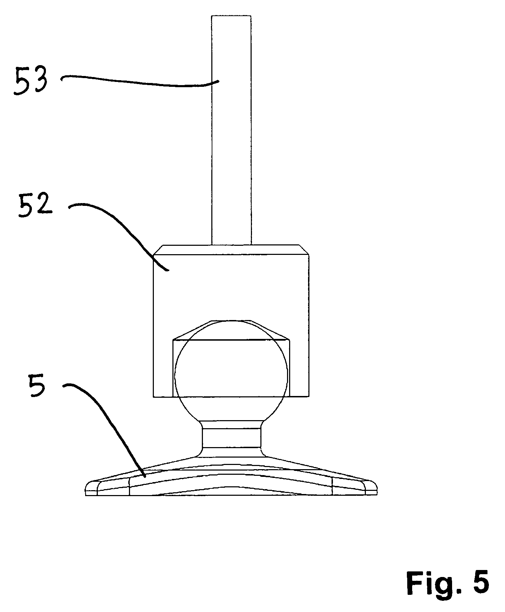

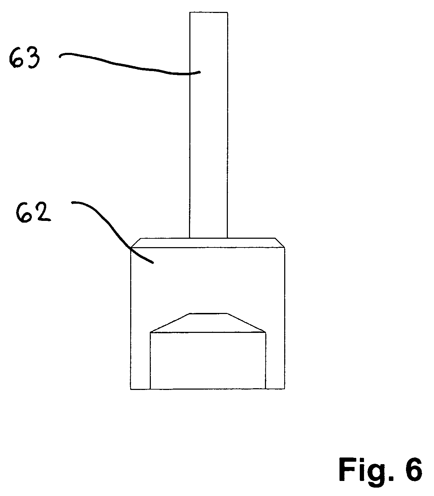

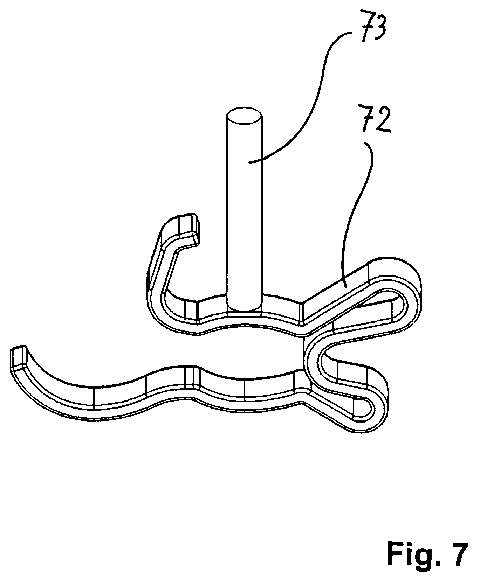

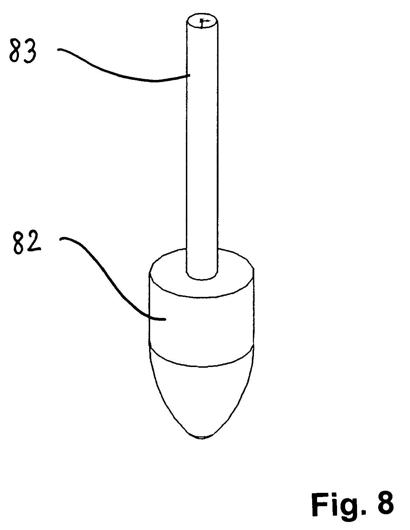

Weitere, unten näher beschriebene Ausführungsformen des zweiten Ankoppelelements 32a; 32b; 42a; 42b; 52; 62; 72; 82 mit einem jeweils schematisch daran angedeuteten Ansatz eines schaftförmigen Mittelteils 33a; 33b; 43a; 43b; 53; 63; 73; 83 sind in den Figuren 3a bis 8 gezeigt.

Die Anordnung 1 zeichnet sich erfindungsgemäß dadurch aus, dass das erste Ankoppelelement 11; 21 geometrisch so gestaltet ist, dass es das Aktor-Endstück 2 mit zwei gegenüber liegenden, im Wesentlichen parallelen Schenkeln 11',11"; 21',21" umgreift, und dass die beiden Schenkel 11',11"; 21',21" jeweils eine Rastereinrichtung aufweisen, die im Zusammenwirken der beiden Schenkel 11',11"; 21',21" ermöglicht, das umgriffene Aktor-Endstück 2a; 2b auf wählbaren diskreten axialen Positionen in einer Richtung parallel zur Schaftachse a des schaftförmigen Mittelteils 13; 23; 33a; 33b; 43a; 43b; 53; 63; 73; 83 zu fixieren.

Die Rastereinrichtungen der Schenkel 11',11"; 21',21" des ersten Ankoppelelements 11; 21 weisen bei in den Figuren 1a, 1b und 2 dargestellten Ausführungsformen auf den im implantierten Zustand am Außenumfang des Aktor-Endstücks 2a; 2b anliegenden Innenflächen jeweils an gegenüberliegenden axialen Positionen der beiden Schenkel 11',11"; 21',21" Einbuchtungen 14; 24 zur Aufnahme des Aktor-Endstücks 2a; 2b auf, die in axialer Richtung äquidistant angeordnet sind.
Das in Fig. 1a gezeigte Aktor-Endstück 2a weist auf seinem Außenumfang längliche Ausbuchtungen 15 auf, die geometrisch so gestaltet sind, dass sie als Gegenstücke zu den Einkerbungen oder Einbuchtungen 14; 24 an den diskreten axialen Positionen der Rastereinrichtungen des ersten Ankoppelelements 11; 21 passen.

Das Aktor-Endstück 2b in Fig. 1b weist dagegen auf seinem Außenumfang azimutal verlaufende Ringnuten 19 auf, die geometrisch so gestaltet sind, dass sie als Gegenstücke zu den Einkerbungen oder Einbuchtungen 14; 24 an den diskreten axialen Positionen der Rastereinrichtungen des ersten Ankoppelelements 11; 21 passen.

Wie in den Figuren 1a, 1b und 2 gut erkennbar, kann der erste Schenkel 11'; 21' des ersten Ankoppelelements 11; 21 in den schaftförmigen Mittelteil 13; 23 des Verbindungselements 10; 20 übergehen und der zweite Schenkel 11"; 21" über ein Verbindungsstück 16; 26 in seitlichem Abstand mit dem ersten Schenkel 11'; 21' verbunden sein, wobei an dem dem Verbindungsstück 16; 26 entgegengesetzten Ende zwischen dem ersten Schenkel 11'; 21' und dem freien Ende des zweiten Schenkels 11"; 21" ein Einführbereich E zum Einführen des Aktor-Endstücks 2a; 2b vorgesehen ist. Das freie Ende des zweiten Schenkels 11"; 21" mündet in eine vom ersten Schenkel 11'; 21' weg in Richtung auf die Außenseite des zweiten Schenkels 11"; 21" schräg nach außen verlaufende Einführhilfe 17; 27 für das Einsetzen des Aktor-Endstücks 2a; 2b in den Einführbereich E des ersten Ankoppelelements 11; 21. Das Verbindungsstück kann 16; 26 zwei Hörner 18',18"; 28',28" aufweisen, die seitlich jeweils in einer Richtung von der Schaftachse a des schaftförmigen Mittelteils 13; 23 weg nach außen ragen.

Bei den in den Figuren 1a und 1b gezeigten Ausführungsformen der Erfindung weist das Verbindungselement 10 jeweils ein Kugelgelenk 3 auf, das im Bereich des schaftförmigen Mittelteils 13 angeordnet ist. Statt des einfachen Kugelgelenks 3 könnte auch eine Gelenkkette verwendet werden, die dem Mittelteil 13 eine noch höhere Flexibilität verleihen würde.

Die in Fig. 2 dargestellte alternative Ausführungsform hingegen zeichnet sich dadurch aus, dass das Verbindungselement 20 mit erstem Ankoppelelement 21, schaftförmigem Mittelteil 23 und zweitem Ankoppelelement 22 insgesamt einstückig aufgebaut ist.

Die Figuren 3a bis 8 schließlich zeigen unterschiedliche Ausgestaltungen des zweiten Ankoppelelements, nämlich als Piston 32a oder Kugel 32b zur Ankopplung an das Innenohr oder als Clip 42a oder als Glocke 42b zur Ankopplung an das Steigbügel-Köpfchen oder als Aufnahmeteil 12; 52 für eine Ω-förmige künstliche Steigbügel-fußplatte 5 oder als Stempel 62 zur Ankopplung an die natürliche Steigbügelfußplatte oder als Klammer 72 zur Ankopplung an den Incus-Fortsatz oder als Zapfen 82 zur Ankopplung an den Incus-Körper.

Bei in der Zeichnung nicht näher dargestellten Ausführungsformen der Erfindung kann eine zusätzliche Masse in Abhängigkeit von einem gewünschten, vorgebbaren Frequenzgang der Schallleitung im Mittelohr am Verbindungselement 10; 20 befestigt sein.

Ebenfalls in der Zeichnung nicht dargestellt sind Ausführungsformen der erfindungsgemäßen Anordnung 1, bei denen zumindest abschnittsweise eine biologisch aktive Beschichtung, insbesondere eine wachstumshemmende und/oder eine wachstumsfördernde und /oder eine antibakteriell wirkende Beschichtung, vorgesehen ist.
Als bevorzugtes Material für das Verbindungselement 10; 20 oder Teile davon, zumindest im Bereich des ersten Ankoppelelements 11; 21 kommt ein Material mit Formgedächtnis (=memory effect), insbesondere eine Nickel-Titan-Legierung, vorzugsweise Nitinol zum Einsatz.

## Patentansprüche

1. Anordnung (1) zum Einstellen und Fixieren der Relativlage zwischen einerseits einem zylinderförmigen Aktor-Endstück (2a; 2b) eines aktiven Hör-Implantats und andererseits einem Glied der Gehörknöchelchenkette oder einem Ankoppelteil zum menschlichen Innenohr, insbesondere zur Perilymphe des labyrinthischen Systems oder zum Endolymphraum, wobei die Anordnung (1) ein Verbindungselement (10; 20) mit einem ersten Ankoppelelement (11; 21) zum Ankoppeln des Verbindungselements (10; 20) an das Aktor-Endstück (2a; 2b), mit einem zweiten Ankoppelelements (12; 22; 32a; 32b; 42a; 42b; 52; 62; 72; 82) zum Ankoppeln des Verbindungselements (10; 20) an den Amboss, den Steigbügel, die Steigbügelfußplatte, das ovale Fenster oder das runde Fenster des menschlichen Mittelohrs sowie mit einem schaftförmigen Mittelteil (13; 23; 33a; 33b; 43a; 43b; 53; 63; 73; 83) zwischen dem ersten Ankoppelelement (11; 21) und dem zweiten Ankoppelelement (12; 22; 32a; 32b; 42a; 42b; 52; 62; 72; 82) umfasst, **dadurch gekennzeichnet,**
**dass** das erste Ankoppelelement (11; 21) geometrisch so gestaltet ist, dass es das zylinderförmige Aktor-Endstück (2a; 2b) mit zwei gegenüber liegenden, im Wesentlichen parallelen Schenkeln (11',11"; 21',21") derart umgreift, dass zusätzlich zu der Verstellbarkeit in Achsrichtung des zylinderförmigen Aktor-Endstücks (2a; 2b) auch eine bequeme längenvariable Einpassung und Fixierung des Aktor-Endstücks (2a; 2b) relativ zum ersten Ankoppelelement (11; 21) in einer Richtung quer zur Achse des zylinderförmigen Aktor-Endstücks (2a; 2b) ermöglicht wird, und dass die beiden Schenkel (11',11"; 21',21") jeweils eine Rastereinrichtung aufweisen, die im Zusammenwirken der beiden Schenkel (11',11"; 21',21") ermöglicht, das umgriffene Aktor-Endstück (2a; 2b) auf wählbaren diskreten axialen Positionen in einer Richtung parallel zur Schaftachse (a) des schaftförmigen Mittelteils (13; 23; 33a; 33b; 43a; 43b; 53; 63; 73; 83) zu fixieren.

2. Anordnung nach Anspruch 1, **dadurch gekennzeichnet, dass** die Rastereinrichtungen der Schenkel (11',11"; 21',21") des ersten Ankoppelelements (11; 21) auf den am Außenumfang des Aktor-Endstücks (2a; 2b) anliegenden Innenflächen jeweils an gegenüberliegenden axialen Positionen der beiden Schenkel (11',11"; 21',21") Einkerbungen oder Einbuchtungen (14; 24) zur Aufnahme des Aktor-Endstücks (2a; 2b) aufweisen.

3. Anordnung nach Anspruch 2, **dadurch gekennzeichnet, dass** die Einkerbungen oder Einbuchtungen (14; 24) auf den Innenflächen der beiden Schenkel (11',11"; 21',21") jeweils in axialer Richtung parallel zur Schaftachse (a) des schaftförmigen Mittelteils (13; 23; 33a; 33b; 43a; 43b; 53; 63; 73; 83) äquidistant angeordnet sind.

4. Anordnung nach Anspruch 2 oder 3 mit einem Aktor-Endstück (2a), **dadurch gekennzeichnet, dass** das Aktor-Endstück (2a) auf seinem Außenumfang Ausbuchtungen (15) aufweist, die geometrisch so gestaltet sind, dass sie als Gegenstücke zu den Einkerbungen oder Einbuchtungen (14; 24) an den diskreten axialen Positionen der Rastereinrichtungen des ersten Ankoppelelements (11; 21) passen.

5. Anordnung nach einem der Ansprüche 2 bis 4 mit einem Aktor-Endstück (2b), **dadurch gekennzeichnet, dass** das Aktor-Endstück (2b) auf seinem Außenumfang azimutal verlaufende Ringnuten (19) aufweist, die geometrisch so gestaltet sind, dass sie als Gegenstücke zu den Einkerbungen oder Einbuchtungen (14; 24) an den diskreten axialen Positionen der Rastereinrichtungen des ersten Ankoppelelements (11; 21) passen.

6. Anordnung nach einem der vorhergehenden Ansprüche, **dadurch gekennzeichnet, dass** ein erster Schenkel (11'; 21') des ersten Ankoppelelements (11; 21) in den schaftförmigen Mittelteil (13; 23; 33a; 33b; 43a; 43b; 53; 63; 73; 83) des Verbindungselements (10; 20) übergeht, dass ein zweiter Schenkel (11"; 21") über ein Verbindungsstück (16; 26) in seitlichem Abstand mit dem ersten Schenkel (11'; 21') verbunden ist, und dass an dem dem Verbindungsstück (16; 26) entgegengesetzten Ende zwischen dem ersten Schenkel (11'; 21') und dem freien Ende des zweiten Schenkels (11"; 21") ein Einführbereich (E) zum Einführen des Aktor-Endstücks (2a; 2b) vorgesehen ist.

7. Anordnung nach Anspruch 6, **dadurch gekennzeichnet, dass** das freie Ende des zweiten Schenkels (11"; 21") in eine vom ersten Schenkel (11'; 21') weg in Richtung auf die Außenseite des zweiten Schenkels (11"; 21") schräg nach außen verlaufende Einführhilfe (17; 27) für das Einsetzen des Aktor-Endstücks (2a; 2b) in den Einführbereich (E) des ersten Ankoppelelements (11; 21) mündet.

8. Anordnung nach Anspruch 6 oder 7, **dadurch gekennzeichnet, dass** das Verbindungsstück (16; 26) am freien, dem schaftförmigen Mittelteil (13; 23; 33a; 33b; 43a; 43b; 53; 63; 73; 83) entgegengesetzten Ende des ersten Ankoppelelements (11; 21) angeordnet ist.

9. Anordnung nach einem der Ansprüche 6 bis 8, **dadurch gekennzeichnet, dass** das Verbindungsstück (16; 26) zwei Hörner (18',18"; 28',28") aufweist, die seitlich jeweils in einer Richtung von der Schaftachse (a) des schaftförmigen Mittelteils (13; 23; 33a; 33b; 43a; 43b; 53; 63; 73; 83) weg nach außen ragen.

10. Anordnung nach einem der Ansprüche 1 bis 9, **dadurch gekennzeichnet, dass** das Verbindungselement (10) ein Kugelgelenk (3) oder eine Gelenkkette aufweist, die insbesondere im Bereich des schaftförmigen Mittelteils (13) angeordnet ist.

11. Anordnung nach einem der Ansprüche 1 bis 9, **dadurch gekennzeichnet, dass** das erste Ankoppelelement (21) und das schaftförmige Mittelteil (23) des Verbindungselements (20) einstückig aufgebaut sind.

12. Anordnung nach Anspruch 11, **dadurch gekennzeichnet, dass** das Verbindungselement (20) mit erstem Ankoppelelement (21), schaftförmigem Mittelteil (23) und zweitem Ankoppelelement (22) insgesamt einstückig aufgebaut ist.

13. Anordnung nach einem der vorhergehenden Ansprüche, **dadurch gekennzeichnet, dass** das Verbindungselement (10; 20) ganz oder teilweise zumindest im Bereich des ersten Ankoppelelements (11; 21) aus einem Material mit Formgedächtnis, insbesondere aus einer Nickel-Titan-Legierung, vorzugsweise aus Nitinol hergestellt ist.

14. Anordnung nach einem der vorhergehenden Ansprüche, **dadurch gekennzeichnet, dass** das zweite Ankoppelelement als Piston (22; 32a) oder Kugel (32b) zur Ankopplung an das Innenohr oder als Clip (42a) oder als Glocke (42b) zur Ankopplung an das Steigbügel-Köpfchen oder als Aufnahmeteil (12; 52) für eine Ωförmige künstliche Steigbügelfußplatte (5) oder als Stempel (62) zur Ankopplung an die natürliche Steigbügelfußplatte oder als Klammer (72) zur Ankopplung an den Incus-Fortsatz oder als Zapfen (82) zur Ankopplung an den Incus-Körper ausgebildet ist.

15. Anordnung nach einem der vorhergehenden Ansprüche, **dadurch gekennzeichnet, dass** mindestens eine zusätzliche Masse in Abhängigkeit von einem gewünschten, vorgebbaren Frequenzgang der Schallleitung im Mittelohr am Verbindungselement (10; 20) befestigt ist.

## Claims

1. Assembly (1) for the adjustment and fixation of the relative position between on the one hand a cylindrical actuator end piece (2a; 2b) of an active hearing implant and on the other hand a link in the ossicular chain or a part connecting to the human inner ear, particularly the perilymph of the labyrinth system or the endolymph area, in which the assembly (1) comprises a connecting element (10; 20) with a first connecting element (11; 21) for connecting the connecting element (10; 20) to the actuator end piece (2a; 2b) and with a second connecting element (12; 22; 32a; 32b; 42a; 42b; 52; 62; 72; 82) for connecting the connecting element (10; 20) to the incus, stapes, stapes footplate, oval window or round window of the human middle ear as well as with a shaft-shaped middle part (13; 23; 33a; 33b; 43a; 43b; 53; 63; 73; 83) between the first connecting element (11; 21) and the second connecting element (12; 22; 32a; 32b; 42a; 42b; 52; 62; 72; 82), **characterised in that** the first connecting element (11; 21) is made geometrically so that it grips the cylindrical actuator end piece (2a; 2b) with two opposing essentially parallel legs (11', 11"; 21', 21") in such a way that, in addition to the cylindrical actuator end piece (2a; 2b) being able to be adjusted in the axial direction, comfortable variable length adjustment and fixation of the actuator end piece (2a; 2b) in relation to the first connecting element (11; 21) in a direction crosswise to the axis of the cylindrical actuator end piece (2a; 2b) is also made possible and that both legs (11', 11"; 21', 21") have a latching device, which working together with both legs (11', 11"; 21', 21") makes it possible to fix the gripped actuator end piece (2a; 2b) at discrete axial positions that may be selected in a direction parallel to the shaft axis (a) of the shaft-shaped middle part (13; 23; 33a; 33b; 43a; 43b; 53; 63; 73; 83).

2. Assembly according to claim 1, **characterised in that** the latching devices of the legs (11', 11"; 21', 21") of the first connecting element (11; 21) have indentations or recesses (14; 24) on the inner surfaces lying on the outer circumference of the actuator end piece (2a; 2b) at opposing axial positions on both legs (11', 11"; 21', 21") for receiving the actuator end piece (2a; 2b).

3. Assembly according to claim 2, **characterised in that** the indentations or recesses (14; 24) on the inner surfaces of both legs (11', 11"; 21', 21") are arranged equidistant in the axial direction parallel to the shaft axis (a) of the shaft-shaped middle part (13; 23; 33a; 33b; 43a; 43b; 53; 63; 73; 83).

4. Assembly according to claim 2 or 3 with an actuator end piece (2a), **characterised in that** the actuator end piece (2a) has protrusions (15) on its outer circumference, which are arranged geometrically so that they fit as counterparts to the indentations or recesses (14; 24) at discrete axial positions on the latching devices of the first connecting element (11; 21).

5. Assembly according to one of claims 2 to 4 with an actuator end piece (2b), **characterised in that** the actuator end piece (2b) has ring grooves (19) running azimuthally on its outer circumference, which are arranged geometrically so that they fit as counterparts to the indentations or recesses (14; 24) at discrete axial positions on the latching devices of the first connecting element (11; 21).

6. Assembly according to one of the previous claims, **characterised in that** a first leg (11'; 21') of the first connecting element (11; 21) passes into the shaft-shaped middle part (13; 23; 33a; 33b; 43a; 43b; 53; 63; 73; 83) of the connecting element (10; 20), a second leg (11"; 21") is connected to the first leg (11'; 21') via a connecting piece (16; 26) at a lateral distance and an insertion area E is provided at the end opposite the connecting piece (16; 26) between the first leg (11'; 21') and the free end of the second leg (11"; 21") for inserting the actuator end piece (2a; 2b).

7. Assembly according to claim 6, **characterised in that** the free end of the second leg (11"; 21") opens into an insertion aid (17; 27) running outwards sloping away from the first leg (11'; 21') in a direction towards the outside of the second leg (11"; 21") for inserting the actuator end piece (2a; 2b) into the insertion area (E) of the first connecting element (11; 21).

8. Assembly according to claim 6 or 7, **characterised in that** the connecting piece (16; 26) is arranged at the free end of the first connecting element (11; 21) opposite the shaft-shaped middle part (13; 23; 33a; 33b; 43a; 43b; 53; 63; 73; 83).

9. Assembly according to one of claims 6 to 8, **characterised in that** the connecting piece (16; 26) has two horns (18', 18"; 28', 28"), which project outwards at the side in a direction away from the shaft axis (a) of the shaft-shaped middle part (13; 23; 33a; 33b; 43a; 43b; 53; 63; 73; 83).

10. Assembly according to one of claims 1 to 9, **characterised in that** the connecting element (10) has a ball joint (3) or a link chain, which is arranged in the area of the shaft-shaped middle part (13) in particular.

11. Assembly according to one of claims 1 to 9, **characterised in that** the first connecting element (21) and the shaft-shaped middle part (23) of the connecting element (20) are made in one piece.

12. Assembly according to claim 11, **characterised in that** the connecting element (20) is made in one piece together with the first connecting element (21), shaft-shaped middle part (23) and second connecting element (22).

13. Assembly according to one of the previous claims, **characterised in that** the connecting element (10; 20) is produced completely or partly from a material with memory effect, particularly from a nickel titanium alloy, preferably from nitinol, at least in the area of the first connecting element (11; 21).

14. Assembly according to one of the previous claims, **characterised in that** the second connecting element is made as a piston (22; 32a) or ball (32b) for connecting to the inner ear or as a clip (42a) or as a bell (42b) for connecting to the stapes head or as a receiving part (12; 52) for an Ω-shaped artificial stapes footplate (5) or as a stamp (62) for connecting to the natural stapes footplate or as a clip (72) for connecting to the incus extension or as a pin (82) for connecting to the incus body.

15. Assembly according to one of the previous claims, **characterised in that** at least one additional mass is fastened to the connecting element (10; 20) in the middle ear depending on a desired, preset frequency response of sound conduction.

## Revendications

1. Agencement (1) pour le réglage et la fixation de la position relative entre d'une part un embout terminal d'actionnement (2a ; 2b) de forme cylindrique d'un implant auditif actif et d'autre part un organe de la chaîne des osselets auditifs ou d'une pièce d'accouplement vers l'oreille interne humaine, en particulier vers la périlymphe du système labyrinthique ou vers l'espace endolymphe, dans lequel l'agencement (1) inclut un élément de liaison (10 ; 20) avec un premier élément de couplage (11 ; 21) pour le couplage de l'élément de liaison (10 ; 20) à l'embout terminal d'actionnement (2a ; 2b), avec un second élément de couplage (12 ; 22 ; 32a ; 32b ; 42a ; 42b ; 52 ; 62 ; 72 ; 82) pour l'accouplement de l'élément de liaison (10 ; 20) à l'enclume, à l'étrier, à la plaque de base de l'étrier, à la fenêtre ovale ou à la fenêtre ronde de l'oreille moyenne humaine, et avec une partie médiane (13 ; 23 ; 33a ; 33b ; 43a ; 43b ; 53 ; 63 ; 73 ; 83) en forme de tige entre le premier élément de couplage (11 ; 21) et le second élément de couplage (12 ; 22 ; 32a ; 32b ; 42a ; 42b ; 52 ; 62 ; 72 ; 82), **caractérisé en ce que** le premier élément de couplage (11 ; 21) est conçu géométriquement de telle façon qu'il enserre l'embout terminal d'actionnement (2a ; 2b) de forme cylindrique avec deux branches mutuellement opposées et essentiellement parallèles (11', 11" ; 21', 21") de telle façon qu'en plus de la possibilité de réglage en direction axiale de l'embout terminal d'actionnement (2a ; 2b) un ajustement confortable et variable en longueur et une fixation de l'embout terminal d'actionnement (2a ; 2b) par rapport au premier élément de couplage (11 ; 21) dans une direction transversale à l'axe de l'embout terminal d'actionnement (2a ; 2b) sont rendus possibles, et **en ce que** les deux branches (11', 11" ; 21', 21") comportent chacune un moyen formant trame qui, en coopération des deux branches (11', 11" ; 21', 21 "), rend possible de fixer l'embout terminal d'actionnement (2a ; 2b) enserré à des positions axiales discrètes susceptibles d'être sélectionnées dans une direction parallèle à l'axe (a) de la partie médiane en forme de tige (13 ; 23 ; 33a ; 33b ; 43a ; 43b ; 53 ; 63 ; 73 ; 83).

2. Agencement selon la revendication 1, **caractérisé en ce que** les moyens formant trame des branches (11', 11" ; 21', 21 ") du premier élément de couplage (11 ; 21) comprennent respectivement, sur les surfaces intérieures appliquées sur la périphérie extérieure de l'embout terminal d'actionnement (2a ; 2b) à des positions axiales opposées des deux branches (11', 11" ; 21', 21 "), des encoches ou des creux (14 ; 24) pour la réception de l'embout terminal d'actionnement (2a ; 2b).

3. Agencement selon la revendication 2, **caractérisé en ce que** les encoches ou les creux (14 ; 24) sont agencés, sur les surfaces intérieures des deux branches (11', 11" ; 21', 21 "), respectivement de manière équidistante en direction axiale parallèlement à l'axe (a) de la partie médiane (13 ; 23 ; 33a ; 33b ; 43a ; 43b ; 53 ; 63 ; 73 ; 83) en forme de tige.

4. Agencement selon la revendication 2 ou 3 avec un embout terminal d'actionnement (2a), **caractérisé en ce que** l'embout terminal d'actionnement (2a) comporte sur sa périphérie extérieure des bosses (15), qui sont conçues sur le plan géométrique de telle façon qu'elles sont adaptées à titre d'éléments antagonistes vis-à-vis des encoches ou des creux (14 ; 24) aux positions axiales discrètes des moyens formant trame du premier élément de couplage (11 ; 21).

5. Agencement selon l'une des revendications 2 à 4 avec un embout terminal d'actionnement (2b), **caractérisé en ce que** l'embout terminal d'actionnement (2b) comporte sur sa périphérie extérieure des rainures annulaires (19) s'étendant en direction azimutale et qui sont conçues sur le plan géométrique de telle façon qu'elles sont adaptées à titre d'éléments antagonistes vis-à-vis des encoches ou des creux (14 ; 24) aux positions axiales discrètes des moyens formant trame du premier élément de couplage (11 ; 21).

6. Agencement selon l'une des revendications précédentes, **caractérisé en ce qu'**une première branche (11' ; 21') du premier élément de couplage (11 ; 21) se transforme dans la partie médiane en forme de tige (13 ; 23 ; 33a ; 33b ; 43a ; 43b ; 53 ; 63 ; 73 ; 83) de l'élément de liaison (10 ; 20), **en ce qu'**une seconde branche (11" ; 21") est reliée, via une pièce de liaison (16 ; 26), à une distance latérale avec la première branche (11' ; 21'), et **en ce qu'**il est prévu une zone d'introduction (E), destinée à l'introduction de l'embout terminal d'actionnement (2a ; 2b), à l'extrémité opposée à la pièce de liaison (16 ; 26) entre la première branche (11' ; 21') et l'extrémité libre de la seconde branche (11" ; 21 ").

7. Agencement selon la revendication 6, **caractérisé en ce que** l'extrémité libre de la seconde branche (11" ; 21 ") débouche dans un auxiliaire d'introduction (17 ; 27), qui s'étend en oblique vers l'extérieur en s'éloignant de la première branche (11'; 21') en direction de la face extérieure de la seconde branche (11" ; 21") pour la mise en place de l'embout terminal d'actionnement (2a ; 2b) dans la zone d'introduction (E) du premier élément de couplage (11 ; 21).

8. Agencement selon la revendication 6 ou 7, **caractérisé en ce que** la pièce de liaison (16 ; 26) est agencée à l'extrémité libre, opposée à la partie médiane en forme de tige (13 ; 23 ; 33a ; 33b ; 43a ; 43b ; 53 ; 63 ; 73 ; 83), du premier élément de couplage (11 ; 21).

9. Agencement selon l'une des revendications 6 à 8, **caractérisé en ce que** la pièce de liaison (16 ; 26) comporte deux cornes (18', 18" ; 28', 28"), qui font saillie latéralement vers l'extérieur respectivement dans une direction en s'éloignant de l'axe (a) de la partie médiane en forme de tige (13 ; 23 ; 33a ; 33b ; 43a ; 43b ; 53 ; 63 ; 73 ; 83).

10. Agencement selon l'une des revendications 1 à 9, **caractérisé en ce que** l'élément de liaison (10) comprend une articulation à rotule (3) ou une chaîne articulée, qui est agencée en particulier dans la région de la partie médiane en forme de tige (13).

11. Agencement selon l'une des revendications 1 à 9, **caractérisé en ce que** le premier élément de couplage (21) et la partie médiane en forme de tige (23) de l'élément de liaison (20) sont réalisés d'une seule pièce.

12. Agencement selon la revendication 11, **caractérisé en ce que** l'élément de liaison (20) est réalisé d'une seule pièce avec le premier élément de couplage (21), avec la partie médiane en forme de tige (23) et avec le second élément de couplage (22) dans l'ensemble.

13. Agencement selon l'une des revendications précédentes, **caractérisé en ce que** l'élément de liaison (10 ; 20) est fabriqué, en totalité ou en partie et au moins dans la région du premier élément de couplage (11 ; 21), en un matériau à mémoire de forme, en particulier un alliage nickel-titane, et de préférence en nitinol.

14. Agencement selon l'une des revendications précédentes, **caractérisé en ce que** le second élément de couplage est réalisé sous forme de piston (22 ; 32a) ou de sphère (32b) pour l'accouplement à l'oreille intérieure, ou bien sous forme de pince (42a) ou de cloche (42b) pour l'accouplement à la tête de l'étrier, ou encore sous forme de pièce de réception (12 ; 52) pour une plaque de base d'étrier prothétique (5) en forme de Q, ou sous forme de poinçon pour l'accouplement à la plaque de base naturelle de l'étrier, ou sous forme d'agrafe (72) pour l'accouplement au prolongement de l'incus, ou de tenon (82) pour l'accouplement au corps de l'incus.

15. Agencement selon l'une des revendications précédentes, **caractérisé en ce qu'**au moins une masse additionnelle est fixée sur l'élément de liaison (10 ; 20) en fonction d'une gamme de fréquence souhaitée prédéterminée dans le conduit auditif dans l'oreille moyenne.
